(19) Europäisches Patentamt
European Patent Office
Office européen des brevets

(11) Veröffentlichungsnummer: **0 538 676 B1**

(12) **EUROPÄISCHE PATENTSCHRIFT**

(45) Veröffentlichungstag der Patentschrift: **28.06.95**

(51) Int. Cl.[6]: **C07C 68/00**, C07C 69/96

(21) Anmeldenummer: **92117145.0**

(22) Anmeldetag: **08.10.92**

# (54) Verfahren zur Herstellung von Dialkylcarbonaten.

(30) Priorität: **21.10.91 DE 4134688**

(43) Veröffentlichungstag der Anmeldung:
**28.04.93 Patentblatt 93/17**

(45) Bekanntmachung des Hinweises auf die Patenterteilung:
**28.06.95 Patentblatt 95/26**

(84) Benannte Vertragsstaaten:
**BE CH DE FR GB IT LI NL**

(56) Entgegenhaltungen:
**EP-A- 0 425 197**

(73) Patentinhaber: **BAYER AG**
**D-51368 Leverkusen (DE)**

(72) Erfinder: **Landscheidt, Heinz, Dr.**
**Liliencronstrasse 6**
**W-4100 Duisburg 1 (DE)**
Erfinder: **Wolters, Erich, Dr.**
**Stenzelbergerstrasse 11**
**W-5000 Köln 41 (DE)**
Erfinder: **Klausener, Alexander, Dr.**
**Weissdornweg 37**
**W-5190 Stolberg (DE)**
Erfinder: **Blank, Heinz-Ulrich, Dr.**
**Am Geusfelde 35**
**W-5068 Odenthal (DE)**
Erfinder: **Birkenstock, Udo, Dr.**
**Schneppersdelle 2**
**W-4030 Ratingen 8 (DE)**

EP 0 538 676 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die vorliegende Erfindung betrifft ein Verfahren zur Herstellung von Dialkylcarbonaten durch Umsetzung von Kohlenmonoxid (CO) mit Alkylnitriten in Gegenwart eines Katalysators aus der Reihe der Platinmetall-Halogenide auf einem Träger aus der Reihe der oxidischen oder hydroxidischen Verbindungen der Elemente der Gruppe Vb des Periodensystems (Vanadium, Niob, Tantal), der Zusätze von Verbindungen weiterer Elemente enthalten kann.

Dialkylcarbonate sind von allgemeiner chemischer Bedeutung. So ist beispielsweise Diethylcarbonat ein ausgezeichnetes Lösungsmittel im mittleren Siedebereich. Des weiteren sind die Dialkylcarbonate ausgezeichnete Carbonylierungs- und Acylierungsreagenzien. Schließlich haben sie große Bedeutung bei der Herstellung von anderen Carbonaten, von Urethanen und von Harnstoffen.

Es ist bekannt, Dialkylcarbonate durch Umsetzungen von Phosgen bzw. von Chlorameisensäurealkylestern mit Alkoholen herzustellen. Es besteht jedoch ein steigendes Interesse daran, den Einsatz des giftigen Phosgens bzw. der davon abgeleiteten Zwischenprodukte, wie der Chlorameisensäureester, durch andere Verfahren abzulösen. Neben Versuchen, Dialkylcarbonate durch Umsetzung von CO mit niederen Alkoholen zu erhalten, sind insbesondere Verfahren wichtig, in denen CO in der Gasphase mit Alkylnitrit an einem Platinmetall-Katalysator umgesetzt werden. Bei solchen Umsetzungen wird neben dem gewünschten Dialkylcarbonat stets das Auftreten von Dialkyloxalat beobachtet.

So ist aus EP 425 197 ein Verfahren bekannt, das gemäß seiner bevorzugten Ausführungsform zu Dialkylcarbonaten des Methanols bzw. Ethanols aus CO und Methyl- bzw. Ethylnitrit in der Gasphase an einem $PdCl_2$-Katalysator auf Aktivkohle führt. Die Selektivitäten zu den gewünschten niederen Dialkylcarbonaten erreichen gemäß dieser EP 425 197, Tabelle 1, Werte bis zu 94 %; jedoch werden als Nebenprodukte stets niedrige Dialkyloxalate und $CO_2$ beobachtet. Bei einer Nacharbeitung konnten darüber hinaus die angegebenen hohen Selektivitäten nur ungenügend reproduziert werden. Die Katalysatoren dieser EP 425 197 enthalten Zusätze von Chloriden unedler Metalle; ein beträchtlicher Zusatz von Chlorwasserstoff, nämlich eine Menge von 1 bis 50 mol-%, bezogen auf das Platinmetall im Katalysator, wird dem System zugesetzt oder ein Teil des Katalysators muß aus dem Reaktor entnommen werden und einer Behandlung mit Chlorwasserstoff unterzogen werden.

Auch in der Zeitschrift für Katalytische Forschung (Dalian, China), Vol. 10 (1), S. 75-78 (1989), wird als Träger für einen $PdCl_2$-haltigen Katalysator ein Träger aus Kohle eingesetzt, um aus CO und Methylnitrit Dimethylcarbonat zu erhalten, wobei jedoch stets nebenher zusätzlich das Dimethyloxalat entsteht.

Ein Pd/Kohle-Katalysator wird auch in Chin. Sci. Bull. 34 (1989), 875-76 für die Herstellung von Dimethylcarbonat aus CO und Methylnitrit erwähnt.

Diese Bevorzugung eines Kohleträgers ist nicht unerwartet, da in Platinum Metals Review 34 (1990), 178-180 unter Bezugnahme auf ältere Literatur davon berichtet wird, daß bei der Umsetzung eines niederen Alkylnitrits mit CO an einem Pd-Katalysator je nach dem Träger verschiedene Hauptprodukte erhalten werden; ein Kohle-Träger ergibt demnach überwiegend die Dialkylcarbonate, während ein oxidischer Träger wie z.B. ein $Al_2O_3$-Träger in der Hauptsache Dialkyloxalate ergibt.

Es wurde nun gefunden, daß oxidische oder hydroxidische Verbindungen der Elemente der Gruppe Vb des Periodensystems als Katalysatorträger nicht nur in unerwarteter Weise zu den Dialkylcarbonaten führt, wenn man CO mit Alkylnitriten umsetzt, sondern es wurde darüber hinaus auch noch eine bedeutende Steigerung der Selektivität zu den gewünschten Dialkylcarbonaten erzielt, die soweit geht, daß neben mehr als 97 % Selektivität, in vielen Fällen mehr als 99 % Selektivität zu diesen Dialkylcarbonaten im allgemeinen überhaupt kein Oxalat nachgewiesen werden kann. Lediglich eine geringe Menge an $CO_2$ kann im Reaktionsgemisch beobachtet werden. Darüber hinaus hat die für nicht möglich gehaltene Verwendung der oxidischen und hydroxidischen Verbindung der Elemente der Gruppe Vb des Periodensystems als Träger den wesentlichen Vorteil einer erhöhten Stabilität und Abriebfestigkeit, verglichen mit einem Trägerkatalysator auf der Basis eines Kohleträgers.

Es wurde ein Verfahren zur Herstellung von Dialkylcarbonaten der Formel

$$O=C(OR)_2 \quad (I),$$

worin

R für geradkettiges oder verzweigtes $C_1$-$C_4$-Alkyl steht,

durch Umsetzung von Kohlenmonoxid (CO) mit Alkylnitriten der Formel

$$RONO \quad (II),$$

worin
R die angegebene Bedeutung hat,
in Anwesenheit oder Abwesenheit eines Inertgases sowie in Anwesenheit oder Abwesenheit des zugrundeliegenden Alkohols ROH sowie in Anwesenheit oder Abwesenheit von NO an einem Platinmetall-Träger-Katalysator bei erhöhter Temperatur in kontinuierlicher Gasphasenreaktion gefunden, das dadurch gekennzeichnet ist, daß als Träger oxidische oder hydroxidische Verbindungen der Elemente der Gruppe Vb des Periodensystems (Mendelejew) eingesetzt werden, das Platinmetall in Form eines Halogenids oder einer Halogenid enthaltenden Komplexverbindung vorliegt, wobei das Platinmetallhalogenid oder der das Platinmetall enthaltende halogenhaltige Komplex in situ im Verfahrensreaktor aus dem Platinmetall oder einer halogenfreien Platinmetallverbindung mit Hilfe von Halogenwasserstoff unter den Reaktionsbedingungen gebildet werden kann, der Katalysator weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet ist und bei einem Volumenverhältnis von Nitrit:CO = 0,1-10:1 und einer Temperatur von 50-150 °C gearbeitet wird, wobei absatzweise oder kontinuierlich Halogenwasserstoff in einer Menge nachgesetzt wird, wie Halogenwasserstoff mit dem Reaktionsgemisch aus dem Reaktor herausgetragen wird.

Die Reaktion im erfindungsgemäßen Verfahren erfolgt im Sinne der nachfolgenden Reaktionsgleichung:

$$CO + 2\ RONO \rightarrow O{:}C(OR)_2 + 2\ NO.$$

Geradkettiges oder verzweigtes Alkyl mit 1-4 C-Atomen ist beispielsweise Methyl, Ethyl, Propyl, Isopropyl, Butyl, Isobutyl, in bevorzugter Weise die genannten n-Alkyle, in besonders bevorzugter Weise Methyl und Ethyl und in ganz besonders bevorzugter Weise Methyl.

Grundsätzlich ist es möglich, von einem Gemisch verschiedener Alkylnitrite auszugehen, wobei jedoch auch ein Gemisch verschiedener Dialkylcarbonate und gegebenenfalls unsymmetrisch substituierte Dialkylcarbonate entstehen. Im Sinne einer einheitlichen Reaktion ist es daher bevorzugt, von nur einem Alkylnitrit auszugehen.

Während es grundsätzlich möglich ist, CO mit einem Alkylnitrit ohne weitere Gemischkomponente umzusetzen, beispielsweise wenn man sich in der Gemischzusammensetzung außerhalb der Explosionsgrenzen befindet, wird vielfach ein Inertgas zur Verdünnung der Reaktionspartner herangezogen. Inertgase sind beispielsweise Edelgase, Stickstoff und Kohlendioxid, bevorzugt Argon, Stickstoff oder Kohlendioxid, besonders bevorzugt Stickstoff und Kohlendioxid. Die Menge des Inertgases beträgt 20-80 Vol.-%, bevorzugt 30-70 Vol.-%, bezogen auf das gesamte in den Reaktor einzuführende Gasvolumen. Das Inertgas und gegebenenfalls darin enthaltene nicht umgesetzte Restmengen der Reaktionspartner kann recyclisiert werden.

Das Volumenverhältnis der Reaktionspartner Nitrit und CO unter sich beträgt 0,1-10:1, bevorzugt 0,2-4:1, besonders bevorzugt 0,3-3:1.

Das umzusetzende Gasgemisch kann weiterhin geringe Mengen an Alkohol ROH, beispielsweise in einer Menge von 0-10 Vol.-% und geringe Mengen an NO, beispielsweise in einer Menge von 0-10 Vol.-%, beides bezogen auf das Gesamtvolumen des einzusetzenden Gasgemisches, enthalten. Solche Zusätze an ROH bzw. NO können etwa aus der Herstellung des Alkylnitrits stammen und beispielsweise mit diesem in das Reaktionsgasgemisch hereingebracht werden.

Der Katalysator für das erfindungsgemäße Verfahren wird auf die oxidische oder hydroxidische Verbindung aus der Reihe der Elemente der Gruppe Vb des Periodensystems (Mendelejew) als Träger aufgebracht; seine Reaktivkomponente besteht im reaktionsbereiten Zustand aus dem Platinmetall-Halogenid oder der Platinmetall-Halogenid enthaltenden Komplexverbindung. Solche Komplexverbindungen sind grundsätzlich bekannt und sind beispielsweise Alkalimetallchlorid-Komplexverbindungen, wie Lithium- oder Natrium-tetrachloropalladat, $Li_2[PdCl_4]$ bzw. $Na_2[PdCl_4]$.

Es hat sich weiterhin gezeigt, daß das Platinmetall-Halogenid bzw. das Platinmetall-Halogenid enthaltende Komplexverbindung in situ im Reaktor aus metallischem Platinmetall oder einer halogenfreien Platinmetall-Verbindung unter den Reaktionsbedingungen, d.h. in Gegenwart des umzusetzenden Gasgemisches, mit Hilfe von Halogenwasserstoff gebildet werden kann. Der Reaktor kann demnach auch mit einem ansonsten vergleichbaren Katalysator gefüllt werden, der das Platinmetall zunächst in metallischer Form enthält oder der mit Hilfe einer halogenfreien Platinmetall-Verbindung hergestellt worden war. Solche halogenfreien Platinmetall-Verbindungen, die hierfür in Frage kommen, sind beispielsweise Platinmetall-nitrate, -propionate, -butyrate, -carbonate, -oxide, -hydroxide oder andere dem Fachmann geläufige.

Elemente der Gruppe der Platinmetalle im Sinne der Erfindung sind Palladium, Platin, Iridium, Ruthenium und Rhodium, bevorzugt Palladium, Ruthenium und Rhodium, besonders bevorzugt Palladium.

Halogenide im Sinne der Erfindung sind Fluorid, Chlorid, Bromid und Iodid, bevorzugt Chlorid und Bromid, besonders bevorzugt Chlorid.

Die Menge des Platinmetall-Halogenids oder der das Platinmetall-Halogenid enthaltenden Komplexverbindung beträgt 0,01-8 Gew.-%, bevorzugt 0,05-4 Gew.-%, gerechnet als Platinmetall und bezogen auf das Gesamtgewicht des Katalysators.

Der Katalysator für das erfindungsgemäße Verfahren ist weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet; in einer bevorzugten Weise liegt ein solcher Zusatz vor. Solche Zusätze liegen in salzartiger oder in metallischer Form der genannten Elemente vor. In ähnlicher Weise, wie dies für das Platinmetall weiter oben beschrieben wurde, bildet sich aus der metallischen Form dieser Zusätze und Halogenwasserstoff unter den Reaktionsbedingungen beispielsweise die Halogenidform solcher Zusätze. In bevorzugter Weise bestehen solche Zusätze aus einer Verbindung von Antimon, Wismut, Aluminium, Vanadium, Niob, Tantal oder einem Gemisch mehrerer von innen. Besonders bevorzugt liegen diese Zusätze als Halogenide von Antimon, Wismut, Aluminium, Vanadium, Niob, Tantal und insbesondere bevorzugt als Chloride von Antimon, Wismut, Aluminium, Vanadium, Niob und Tantal vor.

Zu den hydroxidischen bzw. oxidischen Verbindungen der Metalle der Gruppe Vb des Periodensystems, die erfindungsgemäß als Katalysator-Träger fungieren, sei beispielsweise das Folgende genannt:
Vanadium kann in den Oxidationsstufen +2 bis +5 auftreten, und dementsprechend existieren auch die verschiedensten oxidischen bzw. hydroxidischen Verbindungen als Hydroxide, Oxidhydrate und Oxide. Beispielhaft seien die Systeme $V_2O_5$, $[VO(H_2O)_5]^{2\oplus}$ Anion$^{2\ominus}$, $VO_4^{4\ominus}$ Kation$^{4\oplus}$, $VO_2$, $V_2O_3$ und VO genannt. Die Verbindung $V_2O_5$ ist als das Anhydrid der Orthovanadinsäure $H_3VO_4$ zu betrachten, von der sich über Kondensation die Polyvanadinsäuren ableiten. Hierzu gilt die Reaktionsgleichung:

$$nH_3VO_4 \rightleftharpoons (n-1)H_2O + H_{n+2}V_nO_{3n+1} \quad ,$$

wobei n eine beliebige natürliche Zahl ist. Je nach pH-Bereich sind die verschiedenen davon abgeleiteten Spezies existent, wie z.B.:
$HVO_4^{2\ominus}$, $HV_2O_7^{3\ominus}$, $V_3O_8^{6\ominus}$, $HV_{10}O_{28}^{5\ominus}$, $H_2V_{10}O_{28}^{4\ominus}$,
wobei Anionen dieses Typs beliebige Kationen, etwa $H^{\oplus}$, Alkalimetall-, Erdalkalimetall- oder Edelmetallkationen, in stöchiometrischer Weise so binden, daß eine elektrisch neutrale Verbindung resultiert.

Dieses Verhalten ist nicht auf Vanadium beschränkt, sondern gilt ebenso für die Elemente Niob und Tantal. Es seien hier beispielsweise die Verbindungen $Nb_2O_5$, $Ta_2O_5$, $NbO_2$, $TaO_2$, $Nb_2O_3$, NbO, $Nb(OH)_5$ und $Ta(OH)_5$ genannt. Analog zu den Vanadiumverbindungen treten auch bei Niob und Tantal Polyniob- und Polytantalsäuren in den verschiedensten Kondensationsformen auf.

Die Fähigkeit, höherkondensierte Säuren, die sogenannten Isopolysäuren, zu bilden, kann nun dazu genutzt werden, eine Vielzahl katalytisch aktiver Oxiverbindungen der Elemente V, Nb und Ta im erfindungsgemäßen Sinne zu benutzen. Je nachdem, welcher Entwässerungsgrad eingestellt wird, erhält man oxidische Systeme mit unterschiedlichem Säure-Base-Charakter. Dabei kann es sich um ein- oder mehrmetallische Systeme handeln, in denen die Elemente V, Nb und Ta in folgendem Atomverhältnis vorliegen:

$V_i + Nb_k + Ta_l$,

wobei i, k und l ganze oder gebrochene Zahlen zwischen einschließlich 0 und einschließlich 100 sind mit der Maßgabe, daß die Summe aus diesen Zahlen i, k und l 100 ist. Der Wert 100 besagt, daß das betreffende Element in einem Mengenanteil von 100 Atomprozent und die anderen folglich in einem Mengenanteil von 0 Atomprozent vorliegen. Folgende Aufstellung sei beispielhaft angeführt:

| V | Nb | Ta | Summe (Atomprozent) |
|---|---|---|---|
| 0 | 60 | 40 | 100 |
| 0 | 100 | 0 | 100 |
| 100 | 0 | 0 | 100 |
| 10 | 30 | 60 | 100 |

Aus den genannten oxidischen und hydroxidischen Systemen können durch eine Vielzahl von Präparationen Katalysatoren oder Katalysatorträger erhalten werden. Hier seien beispielhaft genannt:

1. Herstellung von Formkörpern aus den entsprechenden oxidischen Systemen.
2. Einarbeitung zusätzlicher katalytisch aktiver Komponenten, z.B. Salze der Elemente der Gruppe der Platinmetalle und/oder Aktivatoren in die Grundmasse vor der Formgebung.
3. Nachträgliche Beladung der zuvor hergestellten Formkörper durch Tränk-, Tauch- oder Sprühprozesse unter Verwendung von Salzen der Elemente der Gruppe der Platinmetalle und/oder Aktivatoren.
4. Aufrollen und/oder Aufsprühen oxidischer oder hydroxidischer Substanzgemische auf stückige Massen beliebiger Zusammensetzung und Herkunft.
5. Aufrollen und/oder Aufsprühen oxidischer oder hydroxidischer Substanzgemische, die bereits die katalytisch aktiven Salzen der Elemente der Gruppe der Platinmetalle und/oder die Aktivatoren enthalten, auf stückige Massen beliebiger Zusammensetzung und Herkunft.
6. Nachträgliche Beladung gemäß 4. hergestellter Träger durch Tränk-, Tauch- oder Sprühprozesse mit den katalytisch aktiven Salzen der Elemente der Gruppe der Platinmetalle und/oder den Aktivatoren.

Die Menge des Zusatzes beträgt das 0,1-100fache, bevorzugt das 0,2-10fache der Menge an Platinmetall, gerechnet als Metall sowohl für den Zusatz als auch für das Platinmetall.

Die Herstellung eines erfindungsgemäß einzusetzenden Katalysators erfolgt nach Methoden, die dem Fachmann grundsätzlich bekannt sind. So kann der Träger mit einer Lösung einer der genannten Platinmetall-Verbindungen getränkt oder besprüht werden. In gleicher Weise wird mit dem oder den genannten Zusätzen verfahren. Für den Fall, daß das Platinmetall als Metall oder in Form des Carbonats, Oxids oder Hydroxids auf dem Träger fixiert werden soll und erst im Reaktor in der beschriebenen Weise mit Hilfe von Halogenwasserstoff unter den Reaktionsbedingungen zum Platinmetall-Halogenid aktiviert werden soll, kann die aufgetragene Platinmetall-Verbindung in einer dem Fachmann bekannten Weise durch ein geeignetes Reduktionsmittel zum Metall reduziert werden oder durch ein geeignetes Fällungsmittel in das Carbonat, Oxid oder Hydroxid umgewandelt werden.

Es wurde ferner beobachtet, daß es zur Erzielung gleichmäßig hoher Selektivitäten an Dialkylcarbonat vorteilhaft ist, Halogenwasserstoff an den Katalysator im Verlauf seiner Einsatzzeit heranzubringen. Es wurde jedoch weiter beobachtet, daß diese Menge an Halogenwasserstoff wesentlich kleiner sein kann, als sie in der Literatur, wie oben angegeben, beschrieben wird. So ist es lediglich erforderlich, die Menge des mit den Reaktionsprodukten ausgetragenen, der aktivierten Form des Katalysators entstammenden Halogenwasserstoffs zu ersetzen. Diese Menge kann analytisch bestimmt werden. Sie bewegt sich im allgemeinen in einem Bereich von 1-2000 $\mu$g Halogenwasserstoff pro gebildetem g Dialkylcarbonat.

Halogenwasserstoff im Sinne der Erfindung ist Fluorwasserstoff, Chlorwasserstoff, Bromwasserstoff und Iodwasserstoff, bevorzugt Chlorwasserstoff und Bromwasserstoff, besonders bevorzugt Chlorwasserstoff.

Der Halogenwasserstoff kann als solcher gasförmig dem Reaktionsgemisch zudosiert werden. Er kann jedoch auch in gelöster Form in einem der im Reaktionsgemisch vorhandenen Stoffe eindosiert werden, so beispielsweise gelöst in dem dem Alkylnitrit zugrunde liegenden Alkohol.

Katalysatoren der beschriebenen Art haben eine hohe Standzeit (> 200 h). Über die bereits beschriebene mechanische Stabilität und Abriebfestigkeit hinaus behalten sie ihre Aktivität und Selektivität außerordentlich lange.

Die genannten Katalysatoren können mit 700-5 000 l Gemisch der gasförmigen Reaktionspartner pro l Katalysator und pro Stunde belastet werden (GHSV).

Das erfindungsgemäße Verfahren wird bei einer Temperatur von 50-150 °C, bevorzugt 70-120 °C, besonders bevorzugt 70-110 °C, und einem Druck von 0,8-10 bar, bevorzugt 1-7 bar, besonders bevorzugt 1-5 bar, durchgeführt.

Die Herstellung der erfindungsgemäß einzusetzenden Alkylnitrite erfolgt nach bekannten Verfahren, beispielsweise aus dem zugehörigen Alkohol und salpetriger Säure, die etwa in situ aus einem Alkalinitrit und einer Mineralsäure, wie Schwefelsäure, gebildet wird. Das im Verlaufe des erfindungsgemäßen Verfahrens gebildete Stickstoffmonoxid NO kann kontinuierlich mit Sauerstoff und neuem Alkohol zu Alkylnitrit regeneriert werden (DE-OS 38 34 065) und gemeinsam mit nicht umgesetzten Reaktionspartnern recyclisiert werden.

Beispiele

Katalysatorherstellung und Definitionen

Vergleichsbeispiel 1

100 ml Aktivkohle-Granulat wurden in bekannter Weise mit einer Lösung von $PdCl_2$ und $CuCl_2$ in Wasser getränkt, das Produkt bei 80 °C im Vakuum (20 Torr) getrocknet. Der fertige Katalysator enthielt 8 g Pd/l und 8 g Cu/l.

Die Raumzeitausbeute (Space Time Yield STY) in [g/l•h] und für Dimethylcarbonat in den Beispielen berechnet sich nach

$$\frac{m_{Dmc}}{V_{Kat} \cdot t,},$$

wobei $m_{DmC}$ die Menge gebildetes Dimethylcarbonat (DMC), $V_{Kat}$ das Katalysatorvolumen und t die Zeit bedeuten.

Die Selektivität S (%) wird berechnet nach

$$S = \frac{n_{DMC}}{n_{DMC} + 2xn_{ODME} + n_{AME} + n_{FDA}} \times 100 \quad [\%]$$

wobei
$^{n}$DMC = Stoffmenge Dimethylcarbonat
$^{n}$ODME = Stoffmenge Oxalsäuredimethylester
$^{n}$AME = Stoffmenge Ameisensäuremethylester
$^{n}$FDA = Stoffmenge Formaldehhyddimethylacetal
bedeuten.

Beispiel 1

100 ml $Nb(OH)_5$-Körper wurden mit einer wäßrigen $Li_2PdCl_4$-Lösung getränkt und das Produkt bei 80 °C im Vakuum (29 Torr) getrocknet. Der Katalysator enthielt dann 8 g Pd/l.

Beispiel 2

100 ml $Ta(OH)_5$-Körper wurden mit einer wäßrigen $Li_2PdCl_4$-Lösung getränkt und das Produkt bei 80 °C im Vakuum (29 Torr) getrocknet. Der Katalysator enthielt dann 8 g Pd/l.

Beispiel 3

100 ml $V_2O_5$-Körper wurden mit einer wäßrigen $Li_2PdCl_4$-Lösung getränkt und das Produkt bei 80 °C im Vakuum (29 Torr) getrocknet. Der Katalysator enthielt dann 8 g Pd/l.

Versuchsbeschreibungen

Beispiel 4

In einem vertikal aufgestellten Glasrohr (Länge 50 cm, Durchmesser 4 cm) wurden zwischen eine Füllung aus Raschig-Ringen 20 ml des Katalysators aus Beispiel 1 gebracht.

Das Glasrohr wurde auf 90 °C erhitzt und ein Gasgemisch aus 45,0 % $N_2$, 22,5 % MeONO, 22,5 % CO und 10,0 % MeOH geleitet. Die Raumgeschwindigkeit betrug 1000 l/l•h. Das dem Reaktor entströmende Gas wurde auf 5 °C gekühlt und die erhaltene kondensierte Phase mittels Gaschromatographie untersucht.

Die nicht kondensierten Produkte wurden mittels IR-Spektroskopie und Massenspektroskopie erfaßt.

Dimethylcarbonat wurde nach 2 h mit einer STY von 120 g/l•h und Selektivität = 99 % gebildet. Nach 10 Stunden betrug die STY 40 g/l•h und die Selektivität 99 %.

Beispiel 5

Der in Beispiel 4 beschriebene Versuch wurde wiederholt, wobei als Katalysator das in Beispiel 2 beschriebene Produkt eingesetzt wurde.

Dimethylcarbonat wurde nach 2 h mit einer STY von 115 g/l•h und einer Selektivität von 99 % gebildet. Nach 10 Stunden betrug die STY 38 g/l•h und die Selektivität 98,5 %.

Beispiel 6

Der in Beispiel 4 beschriebene Versuch wurde wiederholt, wobei als Katalysator der in Beispiel 3 beschriebene eingesetzt wurde.

Dimethylcarbonat wurde nach 2 h mit einer STY von 105 g/l•h und einer Selektivität von 99 % gebildet. Nach 10 Stunden betrug die STY 35 g/l•h und die Selektivität 98,5 %.

Beispiel 7

100 ml $Nb(OH)_5$-Körper wurden mit einer Lösung von 1 Teil $PdCl_2$ und 4 Teilen $AlCl_3 \cdot 6H_2O$ in Wasser getränkt und das Produkt bei 80 °C im Vakuum (29 Torr) getrocknet. Der Katalysator enthielt dann 16 g Pd/l.

Beispiel 8

In einem vertikal aufgestellten Glasrohr (Länge 50 cm, Durchmesser 4 cm) wurden zwischen zwei Füllungen aus Raschig-Ringen 37,5 ml des Katalysators aus Beispiel 7 angeordnet.

Das Glasrohr wurde auf 90 °C erhitzt und ein Gasgemisch aus 58,0 % $N_2$, 23,2 % MeONO, 10,5 % CO und 8,3 % MeOH sowie 0,037 % HCl geleitet. Die Raumgeschwindigkeit betrug 700 l/l•h.

Das dem Reaktor entströmende Gas wurde auf 5 °C gekühlt und die erhaltene konzentrierte Phase mittels Gaschromatographie untersucht. Die nicht kondensierten Produkte wurden mittels IR-Spektroskopie und Massenspektroskopie erfaßt.

Dimethylcarbonat wurde nach 2 h mit einer STY von 120 g/l•h und Selektivität von 99 % gebildet. Nach 40 h betrug die STY 110 g/l•h und die Selektivität 99 %.

**Patentansprüche**

**1.** Verfahren zur Herstellung von Dialkylcarbonaten der Formel

$O = C(OR)_2$,

worin
R für geradkettiges oder verzweigtes $C_1$-$C_4$-Alkyl steht,
durch Umsetzung von Kohlenmonoxid (CO) mit Alkylnitriten der Formel

RONO,

worin
R die angegebene Bedeutung hat,
in Anwesenheit oder Abwesenheit eines Inertgases sowie in Anwesenheit oder Abwesenheit des zugrundeliegenden Alkohols ROH sowie in Anwesenheit oder Abwesenheit von NO an einem Platinmetall-Träger-Katalysator bei erhöhter Temperatur in kontinuierlicher Gasphasenreaktion, dadurch gekennzeichnet, daß als Träger oxidische und hydroxidische Verbindungen der Elemente der Gruppe Vb des Periodensystems (Mendelejew) eingesetzt werden, das Platinmetall in Form eines Halogenids oder einer Halogenid enthaltenden Komplexverbindung vorliegt, wobei das Platinmetallhalogenid oder der das Platinmetall enthaltende halogenhaltige Komplex in situ im Verfahrensreaktor aus dem Platinmetall oder einer halogenfreien Platinmetallverbindung mit Hilfe von Halogenwasserstoff unter den Reaktionsbedingungen gebildet werden kann, der Katalysator weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet ist und bei einem Volumenverhältnis von Nitrit:CO = 0,1-10:1 und einer Temperatur von 50-150 °C gearbeitet wird, wobei absatzweise oder kontinuierlich Halogenwasserstoff, in einer Menge nachgesetzt wird, wie er mit dem Reaktionsgemisch aus dem Reaktor herausgetragen wird.

**2.** Verfahren nach Anspruch 1, dadurch gekennzeichnet, daß bei 70-120 °C, bevorzugt bei 70-110 °C, gearbeitet wird.

**3.** Verfahren nach Anspruch 1, dadurch gekennzeichnet, daß bei einem Volumenverhältnis Nitrit:CO = 0,2-4:1, bevorzugt 0,3-3:1, gearbeitet wird.

**4.** Verfahren nach Anspruch 1, dadurch gekennzeichnet, daß als Platinmetall eines oder mehrere aus der Gruppe von Palladium, Platin, Iridium, Ruthenium und Rhodium, bevorzugt aus der Gruppe von Palladium, Ruthenium und Rhodium, besonders bevorzugt Palladium eingesetzt wird (werden).

**5.** Verfahren nach Anspruch 1, dadurch gekennzeichnet, daß als Halogenid eines Platinmetall eines oder mehrere aus der Gruppe der Fluoride, Chloride, Bromide und Iodide, bevorzugt aus der Gruppe der Chloride und Bromide, besonders bevorzugt der Chloride eingesetzt wird (werden).

**6.** Verfahren nach Anspruch 1, dadurch gekennzeichnet, daß der Katalysator mit einem Zusatz aus einer Verbindung von Antimon, Wismut, Aluminium, Vanadium, Niob, Tantal oder einem Gemisch mehrerer von ihnen, bevorzugt mit einem Zusatz aus einer Verbindung von Aluminium, ausgerüstet ist.

**7.** Verfahren nach Anspruch 1, dadurch gekennzeichnet, daß in Anwesenheit eines Inertgases gearbeitet wird, wobei das Inertgas 20-80 Vol.-%, bevorzugt 30-70 Vol.-%, des gesamten Gasvolumens beträgt.

**8.** Verfahren nach Anspruch 1, dadurch gekennzeichnet, daß bei einer Katalysatorbelastung von 700-5 000 l Gemisch der gasförmigen Reaktionspartner pro Stunde und pro l Katalysator gearbeitet wird.

**9.** Verfahren nach Anspruch 1, dadurch gekennzeichnet, daß Dimethyl- oder Diethylcarbonat durch Umsetzung von CO mit Methyl- oder Ethylnitrit, bevorzugt Dimethylcarbonat durch Umsetzung von CO mit Methylnitrit hergestellt wird.

**10.** Verfahren nach Anspruch 1, dadurch gekennzeichnet, daß bei einem Druck von 0,8-10 bar, bevorzugt 1-7 bar, besonders bevorzugt 1-5 bar, gearbeitet wird.

## Claims

**1.** Process for the preparation of dialkyl carbonates of the formula

$O=C(OR)_2$,

in which
R represents straight-chain or branched $C_1$-$C_4$-alkyl,
by reaction of carbon monoxide (CO) with alkyl nitrites of the formula

RONO,

in which
R has the meaning given,
in the presence or absence of an inert gas and in the presence or absence of the parent alcohol ROH and in the presence or absence of NO over a supported platinum metal catalyst at elevated temperature in a continuous gas phase reaction, characterised in that the supports used are oxidic or hydroxidic compounds of elements from group Vb of the Periodic Table (Mendeleev), the platinum metal is present in the form of a halide or a halide-containing complex compound, the platinum metal halide or the halogen-containing complex comprising the platinum metal being generable in situ in the

process reactor under the reaction conditions from the platinum metal or a halogen-free platinum metal compound by means of hydrogen halide, the catalyst is furthermore provided with or without an addition of an antimony, bismuth, aluminium, copper, vanadium, niobium, tantalum, tin, iron, cobalt, nickel compound or a mixture of a plurality thereof, and the reaction is carried out at a nitrite:CO volume ratio of 0.1-10:1 and a temperature of 50-150 °C, hydrogen halide being replemished batchwise or continuously at its rate of discharge from the reactor together with the reaction mixture.

**2.** Process according to Claim 1, characterised in that the reaction is carried out at 70-120 °C, preferably at 70-110 °C.

**3.** Process according to Claim 1, characterised in that the reaction is carried out at a nitrite:CO volume ratio of 0.2-4:1, preferably 0.3-3:1.

**4.** Process according to Claim 1, characterised in that the platinum metal comprises one or more from the group consisting of palladium, platinum, iridium, ruthenium and rhodium, preferably from the group consisting of palladium, ruthenium and rhodium, particularly preferably palladium.

**5.** Process according to Claim 1, characterised in that the platinum metal halide comprises one or more from the group consisting of fluorides, chlorides, bromides and iodides, preferably from the group consisting of chlorides and bromides, particularly preferably chlorides.

**6.** Process according to Claim 1, characterised in that the catalyst is provided with an addition of an antimony, bismuth, aluminium, vanadium, niobium or tantalum compound or a mixture of a plurality thereof, preferably with an addition of an aluminium compound.

**7.** Process according to Claim 1, characterised in that the reaction is carried out in the presence of an inert gas, the inert gas being 20-80 % by volume, preferably 30-70 % by volume, of the total gas volume.

**8.** Process according to Claim 1, characterised in that the reaction is carried out with a gas hourly space velocity over the catalyst of 700-5 000 l of the mixture of the gaseous reactants per l of catalyst per hour.

**9.** Process according to Claim 1, characterised in that dimethyl carbonate or diethyl carbonate is prepared by reaction of CO with methyl nitrite or ethyl nitrite, preferably dimethyl carbonate by reaction of CO with methyl nitrite.

**10.** Process according to Claim 1, characterised in that the reaction is carried out at a pressure of 0.8-10 bar, preferably 1-7 bar, particularly preferably 1-5 bar.

## Revendications

**1.** Procédé de préparation de carbonates de dialkyle de formule

$O = C(OR)_2$,

dans laquelle
R représente un groupe alkyle à chaîne droite ou ramifiée en $C_1$-$C_4$, par réaction de l'oxyde de carbone (CO) avec des nitrites d'alkyle de formule

RONO,

dans laquelle
R a les significations indiquées ci-dessus,
en présence ou non d'un gaz inerte et en présence ou non de l'alcool correspondant ROH et en présence ou non de NO sur un catalyseur consistant en un métal de la série du platine sur support à température élevée dans une réaction continue en phase gazeuse, caractérisé en ce que l'on utilise en tant que supports des oxydes et hydroxydes des éléments du groupe Vb de la Classification

Périodique (Mendelejew), le métal de la série du platine est à l'état d'halogénure ou de complexe contenant un halogénure, l'halogénure du métal de la série du platine ou le complexe contenant un tel halogénure pouvant être formé in situ dans le réacteur à partir du métal de la série du platine ou d'un dérivé du métal de la série du platine exempt d'halogène à l'aide d'un halogénure d'hydrogène dans les conditions de la réaction, le catalyseur pouvant en outre le cas échéant contenir une adjonction d'un composé de l'antimoine, du bismuth, de l'aluminium, du cuivre, du vanadium, du niobium, du tantale, de l'étain, du fer, du cobalt, du nickel ou d'un mélange de plusieurs de ces métaux, et on opère à des proportions en volume nitrite/CO = 0,1 à 10 : 1, et à une température de 50 à 150 °C, en introduisant par intermittance ou en continu un halogénure d'hydrogène en quantité correspondant à la quantité évacuée du réacteur avec le mélange de réaction.

**2.** Procédé selon la revendication 1, caractérisé en ce que l'on opère à des températures de 70 à 120 °C, de préférence de 70 à 110 °C.

**3.** Procédé selon la revendication 1, caractérisé en ce que l'on opère à des proportions en volume nitrite/CO = 0,2 à 4 : 1, de préférence 0,3 à 3 : 1.

**4.** Procédé selon la revendication 1, caractérisé en ce que le métal de la série du platine consiste en fait en un ou plusieurs métaux du groupe du palladium, du platine, de l'iridium, du ruthénium et du rhodium, de préférence du groupe du palladium, du ruthénium et du rhodium et tout spécialement en le rhodium.

**5.** Procédé selon la revendication 1, caractérisé en ce que l'halogénure d'un métal de la série du platine consiste en un ou plusieurs halogénures pris dans le groupe formé par les fluorures, les chlorures, les bromures et les iodures, de préférence les chlorures et les bromures et tout spécialement les chlorures.

**6.** Procédé selon la revendication 1, caractérisé en ce que le catalyseur contient une adjonction d'un composé de l'antimoine, du bismuth, de l'aluminium, du vanadium, du niobium, du tantale ou d'un mélange de plusieurs de ces métaux, de préférence une adjonction d'un composé de l'aluminium.

**7.** Procédé selon la revendication 1, caractérisé en ce que l'on opère en présence d'un un gaz inerte en proportion de 20 à 80 % en volume, de préférence de 30 à 70 % en volume par rapport au volume gazeux total.

**8.** Procédé selon la revendication 1, caractérisé en ce que l'on opère avec une charge du catalyseur représentant de 700 à 5000 l du mélange des réactifs gazeux par heure et par litre de catalyseur.

**9.** Procédé selon la revendication 1, caractérisé en ce que l'on prépare le carbonate de diméthyle ou de diéthyle par réaction de CO avec le nitrite de méthyle ou d'éthyle, et de préférence le carbonate de diméthyle par réaction de CO avec le nitrite de méthyle.

**10.** Procédé selon la revendication 1, caractérisé en ce que l'on opère sous une pression de 0,8 à 20 bar, de préférence de 1 à 7 bar et plus spécialement de 1 à 5 bar.